# EUROPEAN PATENT SPECIFICATION

(11) **EP 2 721 996 B1**
(45) Date of publication and mention of the grant of the patent: **29.07.2015**
(21) Application number: 12189294.7
(22) Date of filing: 19.10.2012
(51) Int. Cl.: A61B 5/00, A61B 5/1486, H01L 23/10

(54) **Sealing cap for an connector with an electrical connection**
Verschlusskappe für einen Steckverbinder mit elektrischem Anschluss
Bouchon de fermeture pour connecteur ayant une connexion électrique

(43) Date of publication of application: 23.04.2014
(73) Proprietor: Roche Diagnostics GmbH, 68305 Mannheim (DE); F. Hoffmann-La Roche AG, 4070 Basel (CH)
(72) Inventor: Arnold, Marc, 69517 Gorxheimertal (DE); Thome, Klaus, 68789 St. Leon-Rot (DE)
(74) Representative: Richardt Patentanwälte PartG mbB

(56) References cited:
- US-A1- 2009 124 880
- US-A1- 2011 152 644

## Description

### Field of the invention

The invention relates to medical devices with sealed electrical connections, in particular to a sealing cap for cleaning the electrical connections.

### Background and related art

Medical instruments may comprise multiple components and have various electrical connections between them. The electrical connections may be protected by sealing them within connectors. As an example United States Patent Application Publication US 2011/0152644 describes a protective container for holding reusable diagnostic components.

### Summary

The invention provides for a medical device kit and a method of cleaning a first connector of a medical instrument using a sealing cap in the independent claims. Embodiments are given in the dependent claims.

In one aspect the invention provides for a medical device kit. A medical device kit as used herein encompasses a collection of components which may be assembled to form a medical device or components which are used for maintenance of the medical device. The medical device kit comprises a medical instrument with a first connector. The first connector comprises at least one first electrical connection. The at least one first electrical connection is operable for conducting electricity. The first connector holds or supports the at least one first electrical connection. The first connector further comprises a sealing cavity surrounding the at least one first connector. The sealing cavity is operable for sealing the first electrical connection from fluids or gases. The sealing cavity comprises a sealing surface and a wedging surface. The sealing surface may for instance provide for a smooth surface against which an elastic seal can be pressed to seal the sealing cavity. The wedging surface may be operable for forcing the elastic seal against the sealing surface.

The medical device kit further comprises a sealing cap. The sealing cap comprises a sealing tube with an inner cavity. The sealing tube has a flexible tip region. The wedging surface is operable for forcing the flexible tip against the sealing surface to form a flexible tip seal that seals the at least one first electrical connection within the sealing tube. The flexible tip may for instance by an elastic seal which is forced against the sealing surface by the wedging surface. This embodiment may be beneficial because it provides a means of sealing the first electrical connection with the sealing cap using a wedged surface. The wedged surface exerts a force on the flexible tip region which then seals the sealing cavity. The seal does not depend on the sealing tube being at a fixed diameter. This may enable more efficient cleaning of the medical instrument surrounding the first connector.

In another embodiment the medical device kit further comprises a sensor system with a second connector. The second connector comprises a second electrical connection for each first electrical connection. The second electrical connection is operable for forming an electrical connection with its first electrical connection. The second connector is operable for connecting to the first connector. The second connector comprises a sealing element for forming an O-ring seal on the sealing surface. An O-ring seal as used herein encompasses a seal which is formed by an elastic material that is compressed against a smooth surface. The O-ring seal is formed by a convex elastic surface that is pressed against a sealing surface. This medical device kit may be beneficial because the first connector and the second connector form a sealed electrical connection. At the same time the sealing cap provides a means of efficiently cleaning this O-ring seal.

In another embodiment the sealing cavity further comprises an opening of the sealing cavity. The opening is operable for receiving the sealing cap. For example, the opening may be larger than the sealing tube. The second electrical connection is operable for forming the O-ring seal at a first distance from the opening when the second connector and the first connector are assembled. The flexible tip is operable for forming a flexible seal at a second distance from the opening. The second distance is greater than the first distance. This embodiment is particularly beneficial because the second distance is larger than the first distance. This ensures that the material past the O-ring seal may be cleaned thoroughly when the first electrical connection is cleaned.

In another embodiment the sealing surface has an outer portion. The outer portion is between the opening and the second distance. The sealing tube has an outside surface. The outer portion and the outside surface are operable for forming a cleanable volume. When the sealing cap is positioned or engaged into the first connector the at least one first electrical connection is sealed by the inner cavity and the sealing cavity and the cleanable volume is on the outside.

In another embodiment the sealing surface has an inner portion. The inner portion is located at a distance greater than the second distance. The sealing tube has an inside surface. The inner portion and the inside surface are operable for forming a dry volume.

In another embodiment the sealing cavity further comprises a beveled surface in connection with the sealing surface. The beveled surface forms a widening of the sealing cavity. The widening of the sealing cavity extends a third distance into the sealing cavity. The third distance is less than the first distance.

In another embodiment the at least one first electrical connection is a single first electrical connection. The second electrical connection for each first electrical connection is a single second electrical connection. The first connector and the second connector form a coaxial connector assembly.

In another embodiment the wedging surface has a conical profile. The use of a conical profile may be beneficial because it may be used to put uniform force on the flexible tip region when the sealing cap is used to seal the first connector.

In another embodiment the sealing surface has a profile with two parallel surfaces. That is to say if the first connector is sectioned along the section line the sealing surface will have two parallel surfaces. For instance if the sealing surface is cylindrical and the second is along the axis of the cylinder then the profile will show two parallel surfaces. The same would be true for other types of structures for the sealing surface. For instance an oval shape may be used to construct the cylinder instead of a circle and this would also cause a profile with two parallel surfaces when a section is along the axis of the cylinder.

In another embodiment the flexible tip region comprises an convex surface adapted for contacting the sealing surface. The convex surface may also be a oval shaped sealing surface adapted for contacting the sealing surface. This embodiment may be beneficial because the convex surface may function in a manner similar to that of an O-ring. In some embodiments the flexible tip region may be constructed of any of the standard elastomers used for constructing O-rings. For instance hard silicon or other standard elastomers may be used.

In another embodiment the convex sealing surface is ring-shaped. The convex sealing surface is attached to a distal end of the sealing tube. The sealing tube has an outer diameter. The convex sealing surface has a sealing diameter larger than the outer diameter.

In another embodiment the sealing tube has a circular symmetry.

In another embodiment the sealing tube has an oval-shaped profile.

In another embodiment the first connector comprises a first snap component. The sealing cap comprises a second snap component. The first snap component and the second snap component are operable for forming a snap connection for holding the first connector and the sealing cap together.

In another embodiment the sensor system is a transcutaneous sensor system. The medical instrument is a control part of the transcutaneous sensor system.

In another aspect the invention provides for a method of cleaning a first connector of a medical instrument using a sealing cap. The first connector and the sealing cap may be according to an embodiment of the invention. For instance the first connector comprises at least one first electrical connection. The first connector further comprises a sealing cavity surrounding the at least one first electrical connection. The sealing cavity comprises a sealing surface and a wedging surface. The sealing cap comprises a sealing tube with an inner cavity. The sealing tube has a flexible tip region. The wedging surface is operable for forcing the flexible tip against the sealing surface to form a flexible tip seal that seals the at least one first electrical connection with the sealing tube. The method comprises the step of receiving the first connector assembled to a second connector. The second connector comprises a second connector for each first electrical connection. The second electrical connection is operable for forming an electrical connection with its first electrical connection. The second connector is operable for connecting to the first connector. The second connector comprises an O-ring seal for forming an O-ring seal on the sealing surface. The sealing cavity further comprises an opening of the sealing cavity. The opening is larger than the sealing tube. The O-ring is operable for forming the O-ring seal at a first distance from the opening when the second connector and the first connector are assembled. The flexible tip is operable for forming the flexible tip seal at a second distance from the opening. The second distance is greater than the first distance. The method further comprises removing the second connector from the first connector. The method further comprises installing the sealing cap into the first connector. The method further comprises cleaning the second connector with a cleaning fluid. The method further comprises removing the sealing cap. This embodiment may be particularly beneficial because the area of the sealing surface around where the O-ring seal is formed may be cleaned.

It is understood that one or more of the aforementioned embodiments of the invention may be combined as long as the combined embodiments are not mutually exclusive.

### Brief description of the drawings

In the following embodiments of the invention are explained in greater detail, by way of example only, making reference to the drawings in which:
Fig. 1 shows connector mounted in a medical instrument housing;
Fig. 2 shows an embodiment of a first connector assembled with a second electrical connector;
Fig. 3 shows Fig. 2 after the second connector has been removed from the first electrical connector;
Fig. 4 illustrates a sealing cap;
Fig. 5 shows the sealing cap being inserted into the first connector of Fig. 3;
Fig. 6 shows the arrangement of Fig. 5 after the sealing cap has been inserted into the first connector;
Fig. 7 shows the first connector of Fig. 2 and an alternative embodiment of a sealing cap;
Fig. 8 shows a flow diagram which illustrates a method according to an embodiment; and
Fig. 9 schematically illustrates, in an exemplary fashion, an embodiment of a transcutaneous sensor system for detecting at least one analyte in a bodily fluid.

### Detailed description

Like numbered elements in these figures are either equivalent elements or perform the same function. Elements which have been discussed previously will not necessarily be discussed in later figures if the function is equivalent.

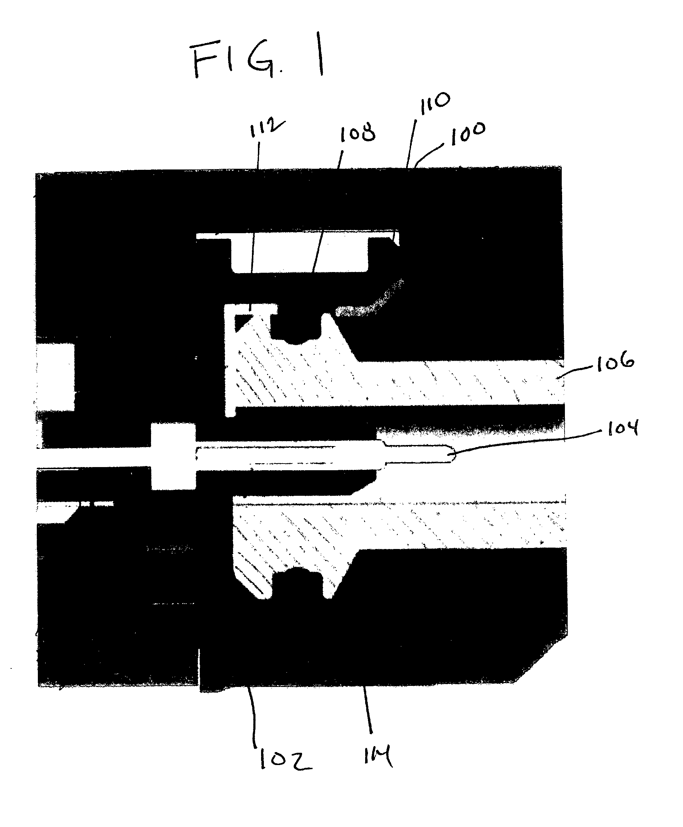

Fig. 1 shows an connector 102 mounted in a medical instrument housing 100. The connector 102 comprises an electrical connection 104. The connector 102 is used to later contrast with embodiments of the invention. However, this connector 102 shows several features which are present in the embodiments of the invention. As such Fig. 1 is not considered to be prior art. Fig. 1 is provided to aid in illustrating the benefits of the embodiments. There is an insert 106 which has an O-ring 108. This O-ring 108 seals the connector 102. The region 110 is a surface which is able to be cleaned 110. The surface 112 is not cleanable. As the insert 106 is pressed into the connector the O-ring 108 rubs against a sealing surface 114. The O-ring 108 is always in compression against the sealing surface 114. This makes it impossible to clean the non-cleanable surface 112. If a different insert 106 is used and the O-ring is placed further into the connector the O-ring 108 would simply drag the material deeper along the sealing surface 114.

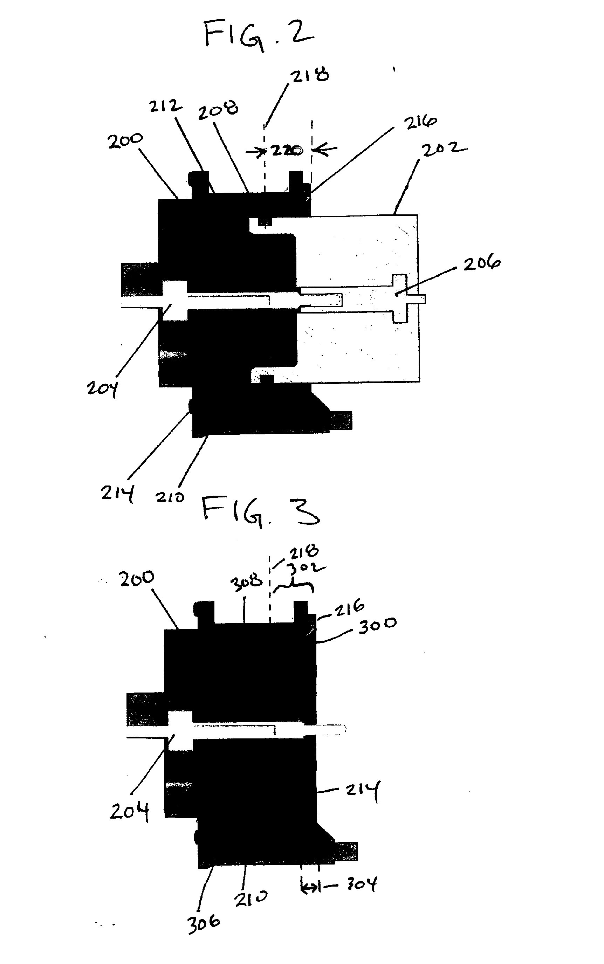

Fig. 2 shows an embodiment of a first connector 200 assembled with a second connector 202. The first connector 200 comprises a first electrical connection 204. The second connector 202 comprises a second electrical connection 206. In other embodiments there may be additional first electrical connections and second electrical connections. The connectors as used herein encompass a housing or mechanical support for the electrical connections. The electrical connections are adapted for conducting electricity. The connectors are mechanical parts. In Fig. 2 it can be shown that the first electrical connection 204 forms an electrical path with the second electrical connection 206.

The second connector 202 has an O-ring 208 which forms an O-ring seal 218 on a sealing surface 210. The O-ring may be another type of sealing element also. The connector 200 has a wedging surface 214. The wedging surface 214 and the sealing surface 210 form a sealing cavity 210. It can be seen that the O-ring seal 218 seals the sealing cavity 212 and protects the first electrical connection 204 and the second electrical connection 206. For instance this would seal them against gases and/or fluids such as bodily fluids. The sealing cavity 212 has an opening 216 that is flush with the surface 216. The O-ring seal 218 is located a first distance 220 from the opening 216. If the first connector 200 and the second connector 202 were immersed in a fluid the sealing surface 210 which is less than the first distance 220 would become contaminated.

In Fig. 2, a single first electrical connection 204 and a single second electrical connection 206 is shown. Fig. 2 is intended to be represent the first 204 and second 206 electrical connection generally. It is understood that other types of electrical connections and a larger number of electrical connections could be used in place of those explicitly shown in Fig. 2.

In Fig. 3 the second connector 200 has been removed from the first connector 200. The opening 216 has a bezelled surface 300. The bezel 300 extends a third distance 304 into the opening 216. The dashed line 218 indicates where the O-ring seal was in Fig. 2. The region 302 of the sealing surface 210 would be contaminated. It may be desirable to clean the region 302. In order to use a liquid solvent or other means to clean the dirty region 302 the first electrical connection 204 needs to be protected. The view of the connector in Figs. 2 and 3 is cross-sectional. It can be seen that the sealing surface 210 has a first parallel surface 306 and a second parallel surface 308 when viewed in cross-section. The wedging surface 214 has a conical profile.

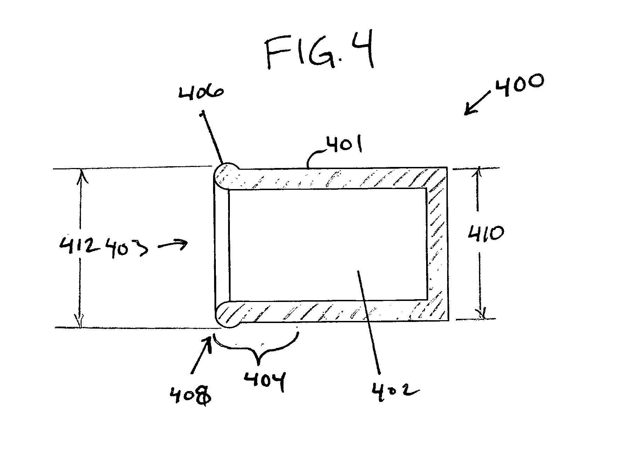

In Fig. 4 an embodiment of a sealing cap 400 is shown. The sealing cap 400 has a sealing tube that is hollow and has an inner cavity 402. The inner cavity 402 is accessible by an opening 403. The sealing cap 400 has a flexible tip region 404. The flexible tip region 404 is operable for being deformed. At the opening 403 there is a distal end 408 to the sealing cap. The sealing cap 400 has a convex sealing surface 406. The convex sealing surface 406 is operable for forming a seal against the sealing surface 210 of Figs. 2 and 3. The sealing cap 400 has an outer diameter 410. At the distal end 408 the convex sealing surface 406 has a sealing diameter 412. The outer diameter 410 is less than the sealing diameter 412.

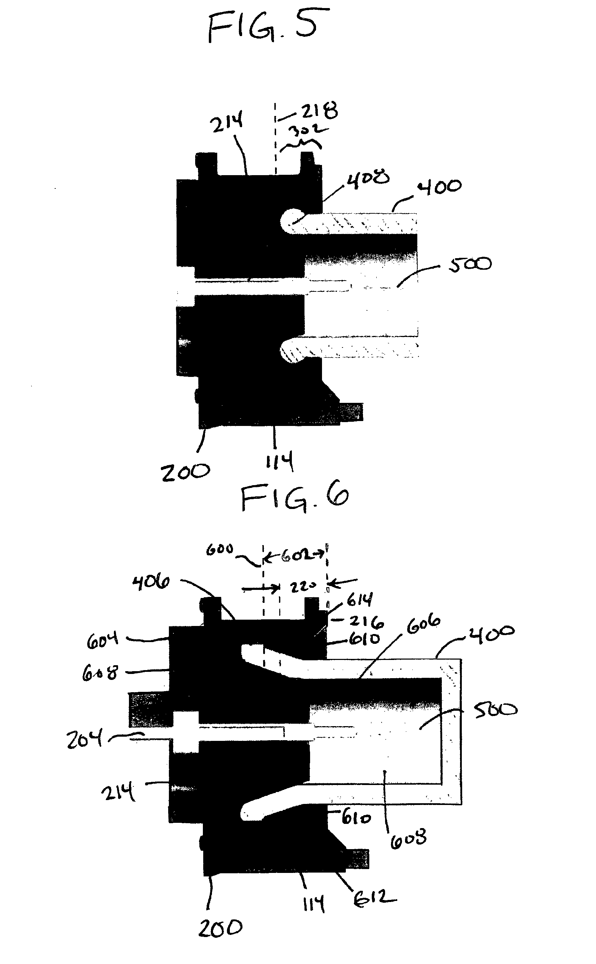

Fig. 5 shows the sealing cap 400 being inserted into the first connector 200. The arrow 500 indicates the direction of travel of the sealing cap 400. It can be seen that the distal end 408 is first contacting the wedging surface 214. As the sealing cap 400 is driven further in direction 500 the wedging surface 214 will exert force on the distal end 408. This will push the distal end 408 in the direction of the sealing surface 114. The dimensions of the sealing cap 400 are chosen such that the distal end 408 does not contact the sealing surface 114 until it has passed the first distance 218. This means that the distal end 408 does not contact the dirty region 302 and itself does not become contaminated.

Fig. 6 shows the sealing cap 400 after it has been inserted into the first connector 200. The wedging surface 214 has pressed the distal tip 408 such that the convex sealing surface 406 is pressed against the sealing surface 114. The dashed line 600 indicates the location of the flexible tip seal 600. The second distance 602 is between the flexible tip seal 600 and the surface 216 indicating the position of the opening. The first distance 220 is less than the second distance 602. The flexible sealing cap was used to form a seal beyond the dirty region 308 without becoming contaminated itself. There is an inner portion of the sealing surface 114. There is also an inside surface 606 of the sealing cap 400. The inner portion 604 and the inside surface 606 form a dry volume 608. There is a trapped volume that forms part of the dry volume 608 and also the region surrounding the first electrical connection. The first electrical connection 204 is protected and the entire dirty region 302 may be cleaned. When the sealing cap 400 was removed the entire first connector 200 will be cleaned. This particular implementation of the sealing cap 400 and the first connector 200 prevents fluid from being trapped when the cleaning process is being performed. This prevents fluid from accidentally being dripped or contaminating the electrical connection 204.

Looking at Fig. 6 it can be seen that there is a region of the sealing cavity 216 that is outside of the dry volume 608. This volume is labeled 610 and is a cleanable volume. The cleanable volume 610 is formed by an outside surface 612 of the sealing cap 400 and an outer portion of the sealing surface 114. The outer portion 614 of the sealing surface 114 is the area of the sealing surface 114 between the opening 212 and the second distance 602. This cleanable surface of the sealing surface 114 extends beyond the first distance 220 where the original O-ring seal was formed.

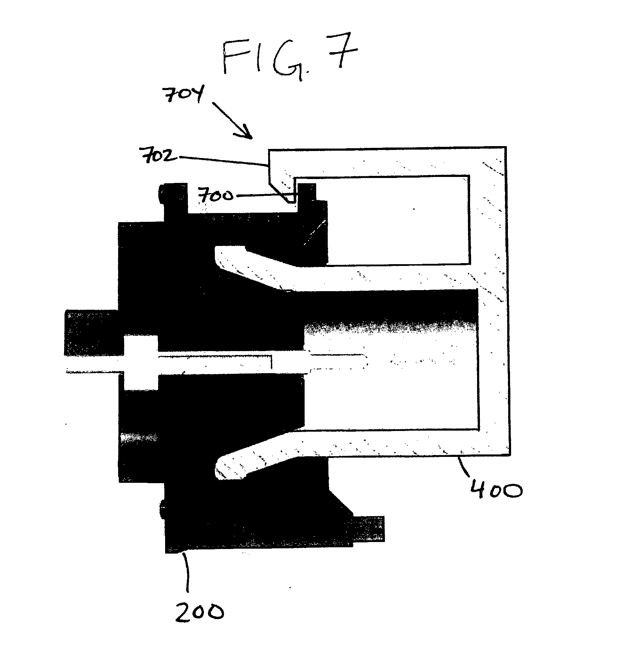

Fig. 7 shows the first connector 200 and an alternative embodiment of a sealing cap 400. The first connector 200 has a first snap component 700. The sealing cap 400 has an extension built out of it with a second snap component 702. The first snap component 700 and the second snap component 702 form a snap connection 704 for holding the sealing cap 400 in place. The embodiment shown in Fig .7 is exemplary and there are large varieties of ways in which a snap connection can be implemented to connect the first connector 200 to the sealing cap 400.

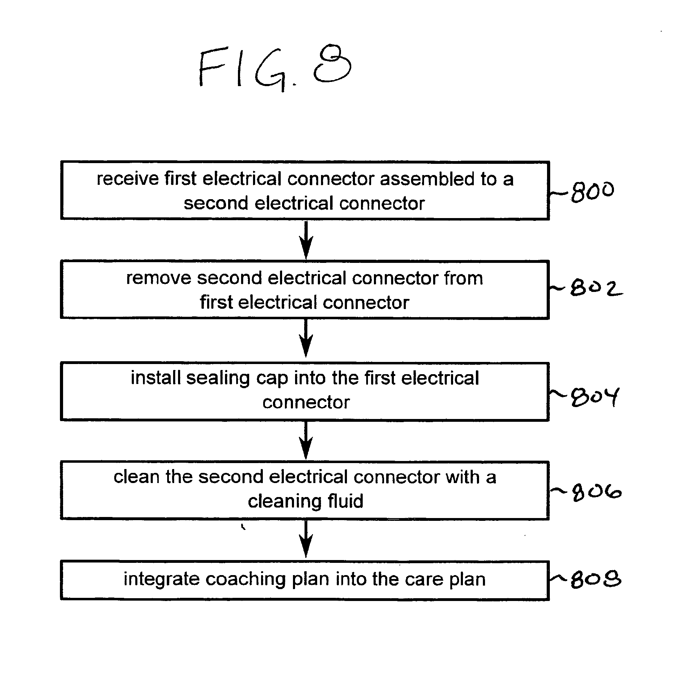

Fig. 8 shows a flow diagram which illustrates a method according to an embodiment. First in step 800 a first connector assembled to a second connector is received. The second connector may be to a sensor system. The second connector comprises an O-ring seal for forming an O-ring seal on a sealing surface of the first connector. Next in step 802 a second connector is removed from the first connector. The first connector and the second connector are according to an embodiment of the invention. Next in step 804 a sealing cap is installed into the first connector. The sealing cap is according to an embodiment of the invention. Next in step 806 the second connector is cleaned with a cleaning fluid. The sealing cap protects the electrical connection of the second connector. Finally in step 808 the sealing cap is removed from the first connector.

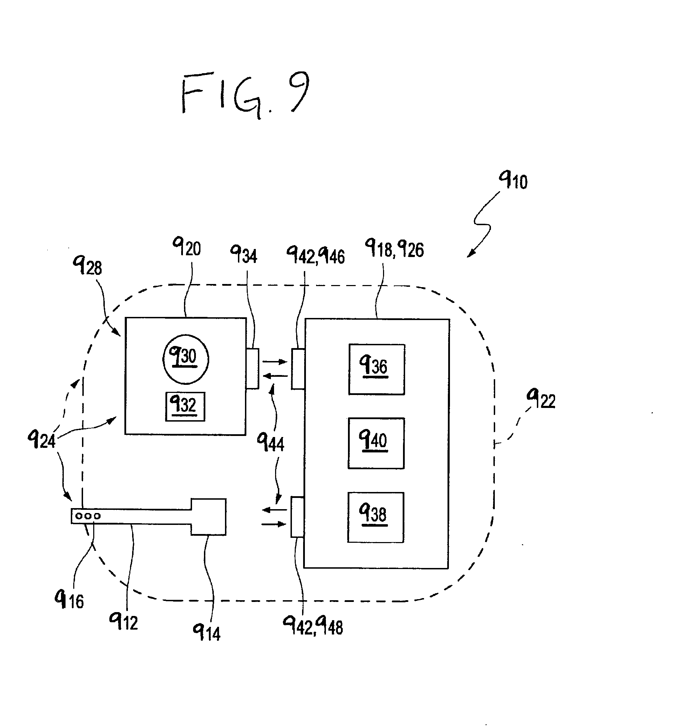

Fig. 9 schematically illustrates, in an exemplary fashion, an embodiment of a transcutaneous sensor system 910 for detecting at least one analyte in a bodily fluid. By way of example, the transcutaneous sensor system 910 in the illustrated embodiment can correspond to the transcutaneous sensor system described in US Pat. Pub. No. 2008/0242962 A1 and/or US Pat. Pub. No 2011/0152644 A1. In the illustrated exemplary embodiment, the transcutaneous sensor system 910 comprises a transcutaneous sensor 912 with a second sensor connector 914 and a sensor region 916 that can be introduced into the body tissue. The second sensor connector 914 may be equivalent to the second connector in Fig. 2. The transcutaneous sensor system 910 also includes a reusable control part 918 and a control component 920, more particularly a disposable control component. The disposable control component can be designed for a single use, for example, for merely a single measurement period. However, other control component designs may also be employed. The control component 920, the transcutaneous sensor 912 and an attachment element 922, such as a plaster for adhesion onto a skin surface, and, if necessary, further components such as, for example, housing components, together form a so-called disposable 924. The disposable 924 is a disposable part, whereas the reusable control part forms a so-called reusable, that is to say, a reusable part. The control component 920 is often also referred to as a bodymount 928.

In the illustrated embodiment, the control component 920 comprises an electrical energy storage device 930 and/or a data storage medium 932 in an exemplary fashion. By way of example, the data storage medium 932 can be designed as a non-volatile data storage medium, for example as a ROM and/or an EEPROM and/or a flash EPROM. In the illustrated embodiment, the control component 920 also includes a second control connector 934. In some embodiments the second control connector is equivalent to the second connector 202 in Fig. 2.

In the illustrated embodiment, the reusable control part 918, in turn, comprises a data-processing device in the form of a microcontroller 936, actuation and evaluation electronics 938 for actuating the transcutaneous sensor 912, and a telemetry component 940 for wireless communication of measurement results. The telemetry component 940 can communicate, for example, with a data manager (not illustrated in Fig. 9), which likewise can optionally be a component of the transcutaneous sensor system 910. Furthermore, the reusable control part 918 can also include one or more storage media, which can also be volatile storage media and/or non-volatile storage media. Whereas the data storage medium 932 in the control component 920 is advantageously used for permanently storing charge information relating to the sensor element 912, for example, producer-specific and/or production-specific information, such as calibration information, the data storage medium of the reusable control part 918, which can, for example, be a component of the microcontroller 936, can be used for storing measurement results, which can subsequently be transmitted to the data manager via the telemetry component 940.

Accordingly, the reusable control part 918 can comprise at least one connector 942, which has an exemplary two-part design in this embodiment and can form a connection 944. In the illustrated embodiment, the connector 942 comprises a first control connector 946 for connecting it to the second control connector 934 and a first sensor connector 948 for connection to the second sensor connector 914. The first sensor connector 948 may be equivalent to the first connector 200 in Fig. 2. The first control connector may be equivalent to the first connector 200 in Fig. 2 in some embodiments. Alternative embodiments are also possible.

### List of reference numerals

- 100: medical instrument housing
- 102: connector
- 104: electrical connection
- 106: insert
- 108: O-ring
- 110: cleanable surface
- 112: non-cleanable surface
- 114: sealing surface
- 200: first connector
- 202: second connector
- 204: first electrical connection
- 206: second electrical connection
- 208: O-ring
- 210: sealing surface
- 212: sealing cavity
- 214: wedging surface
- 216: opening
- 218: location of O-ring seal
- 220: first distance
- 300: beveled surface
- 302: dirty region
- 304: third distance
- 306: first parallel surface
- 308: second parallel surface
- 400: sealing cap
- 402: inner cavity
- 403: opening
- 404: flexible tip region
- 406: convex sealing surface
- 408: distal end
- 410: outer diameter
- 412: sealing diameter
- 500: inserting sealing cap
- 600: location of flexible tip seal
- 602: second distance
- 604: inner portion
- 606: inside surface
- 608: dry volume
- 610: cleanable volume
- 612: outside surface
- 614: outer portion
- 700: first snap component
- 702: second snap component
- 704: snap connection
- 910: transcutaneous sensor system
- 912: transcutaneous sensor
- 914: second sensor connector
- 916: sensor region
- 918: reusable control part
- 920: control component
- 922: an attachment element
- 924: disposable
- 928: bodymount
- 930: electrical energy storage device
- 932: data storage medium
- 934: second control connector
- 936: microcontroller
- 938: evaluation electronics
- 940: telemetry component
- 942: at least one connector
- 944: connection
- 946: first control connector
- 948: first sensor connector

## Claims

1. A medical device kit comprising:
- a medical instrument (918) with a first connector (200, 946, 948), wherein the first connector comprises at least one first electrical connection (204), wherein the first connector further comprises a sealing cavity (212) at least partially surrounding the at least one first electrical connection, wherein the sealing cavity comprises a sealing surface (114) and a wedging surface (214); and
- a sealing cap (400), wherein the sealing cap comprises a sealing tube (401) with an inner cavity (402), wherein the sealing tube has a flexible tip region (404), wherein the wedging surface is operable for forcing the flexible tip against the sealing surface to form a flexible tip seal (600) that seals the at least one first electrical connection within the sealing tube.

2. The medical device kit of claim 1, wherein the medical device kit further comprises a sensor system (916) with a second connector (202, 914), wherein the second connector comprises a second electrical connection (206) for each first electrical connection, wherein the second electrical connection is operable for forming an electrical connection with its first electrical connection, wherein the second connector is operable for connecting to the first connector, wherein the second connector comprises a sealing element (208) for forming an O-ring seal (218) on the sealing surface.

3. The medical device kit of claim 2, wherein the sealing cavity further comprises an opening (216) of the sealing cavity, wherein the opening is operable for receiving the sealing tube, wherein the second connector is operable for forming the O-ring seal at a first distance (220) from the opening when the second connector and the first connector are assembled, wherein the flexible tip is operable for forming the flexible tip seal at a second distance (602) from the opening, and wherein the second distance is greater than the first distance.

4. The medical device kit of claim 3, wherein an outer portion (614) of the sealing surface between the opening and the second distance and an outside surface (612) of the sealing tube are operable for forming a cleanable volume (610).

5. The medical device kit of claim 3 or 4, wherein an inner portion (604) of the sealing surface located at a distance greater than the second distance and an inside surface (606) of the sealing tube are operable for forming a dry volume (608).

6. The medical device kit of claim 3, 4, or 5, wherein the sealing cavity further comprises a beveled surface (300) in connection with the sealing surface, wherein the beveled surface forms a widening of the sealing cavity, wherein the widening of the sealing cavity extends a third distance (304) into the sealing cavity, wherein the third distance is less than the first distance.

7. The medical device kit of claim 2, 3, 4, 5, or 6, wherein the at least one first electrical connection is a single first electrical connection, wherein the second electrical connection for each first electrical connection is a single second electrical connection, wherein the first connector and the second connector form a coaxial connector assembly.

8. The medical device kit of any one of the preceding claims, wherein the wedging surface has a conical profile (214).

9. The medical device kit of any one of the preceding claims, wherein the sealing surface has a profile with two parallel surfaces (306, 308).

10. The medical device kit of any one of the preceding claims, wherein the flexible tip region comprises a convex surface (406) adapted for contacting the sealing surface.

11. The medical device kit of any one of the preceding claims, wherein the convex surface is ring shaped, and wherein the convex surface is attached to a distal end (408) of the sealing tube, wherein the sealing tube has an outer diameter (410), wherein the convex surface has a sealing diameter (412) larger than the outer diameter.

12. The medical device kit of any one of the preceding claims, wherein the sealing tube has any one of the following: a circular symmetry and oval shaped profile.

13. The medical device kit of any one of the preceding claims, wherein the first connector comprises a first snap component (700), wherein the sealing cap comprises a second snap component (702), wherein the first snap component and the second snap component are operable for forming a snap connection (704) for holding the first connector and the sealing cap together.

14. The medical device kit of any one of the preceding claims, wherein the sensor system is a transcutaneous sensor (916), and wherein the medical instrument is a control part (918) of a transcutaneous sensor system (910).

15. A method of cleaning a first connector (200, 946, 948) of a medical instrument (918) using a sealing cap (400), wherein the first connector comprises at least one first electrical connection (204), wherein the first connector further comprises a sealing cavity (212) at least partially surrounding the at least one first electrical connection, wherein the sealing cavity comprises a sealing surface (114) and a wedging surface (214), wherein the sealing cap comprises a sealing tube (401) with an inner cavity (402), wherein the sealing tube has a flexible tip region (404), wherein the wedging surface is operable for forcing the flexible tip against the sealing surface to form a flexible tip seal (600) that seals the at least one first electrical connection within the sealing tube, wherein the method comprises the steps of:
- receiving (800) the first connector assembled to a second connector (202),
wherein the second connector comprises a second electrical connection (206) for each first electrical connection, wherein the second electrical connection is operable for forming an electrical connection with its first electrical connection, wherein the second connector is operable for connecting to the first connector, wherein the second connector comprises a sealing element (208) for forming an O-ring seal (218) on the sealing surface, wherein the sealing cavity further comprises an opening (216) of the sealing cavity, wherein the opening is operable for receiving the sealing tube, wherein the sealing element is operable for forming the O-ring seal at a first distance (220) from the opening when the second connector and the first connector are assembled, wherein the flexible tip is operable for forming the flexible tip seal at a second distance (602) from the opening, and wherein the second distance is greater than the first distance;
- removing (802) the second connector from the first connector;
- installing (804) the sealing cap into the first connector;
- cleaning (806) the second connector with a cleaning fluid; and
- removing (808) the sealing cap from the first connector.

## Patentansprüche

1. Medizinisches Geräteset, umfassend:
- ein medizinisches Instrument (918) mit einem ersten Verbinder (200, 946, 948), wobei der erste Verbinder wenigstens eine erste elektrische Verbindung (204) aufweist, wobei der erste Verbinder ferner einen Dichtungshohlraum (212) aufweist, der die wenigstens eine erste elektrische Verbindung wenigstens teilweise umgibt, wobei der Dichtungshohlraum eine Dichtfläche (114) und eine Einklemmfläche (214) aufweist, und
- eine Dichtungskappe (400), wobei die Dichtungskappe eine Dichtungshülse (401) mit einem inneren Hohlraum (402) aufweist, wobei die Dichtungshülse eine flexible Spitzenregion (404) hat, wobei die Einklemmfläche funktionell ist zum Zwingen der flexiblen Spitze gegen die Dichtfläche, um eine flexible Spitzenabdichtung (600) zu bilden, welche die wenigstens eine erste elektrische Verbindung innerhalb der Dichtungshülse dicht einschließt.

2. Medizinisches Geräteset nach Anspruch 1, wobei das medizinische Geräteset ferner ein Sensorsystem (916) mit einem zweiten Verbinder (202, 914) aufweist, wobei der zweite Verbinder für jede erste elektrische Verbindung eine zweite elektrische Verbindung (206) aufweist, wobei die zweite elektrische Verbindung zum Bilden einer elektrischen Verbindung mit ihrer ersten elektrischen Verbindung funktionell ist, wobei der zweite Verbinder zum Verbinden mit dem ersten Verbinder funktionell ist, wobei der zweite Verbinder ein Dichtungselement (208) zum Bilden eines O-Dichtrings (218) an der Dichtfläche aufweist.

3. Medizinisches Geräteset nach Anspruch 2, wobei der Dichtungshohlraum ferner eine Öffnung (216) des Dichtungshohlraums aufweist, wobei die Öffnung zum Aufnehmen der Dichtungshülse funktionell ist, wobei der zweite Verbinder funktionell ist zum Bilden des O-Dichtrings in einem ersten Abstand (220) von der Öffnung, wenn der zweite Verbinder und der erste Verbinder zusammengefügt werden, wobei die flexible Spitze zum Bilden der flexiblen Spitzenabdichtung in einem zweiten Abstand (602) von der Öffnung funktionell ist und wobei der zweite Abstand größer als der erste Abstand ist.

4. Medizinisches Geräteset nach Anspruch 3, wobei ein Außenteil (614) der Dichtfläche zwischen der Öffnung und dem zweiten Abstand und eine Außenfläche (612) der Dichtungshülse funktionell sind zum Bilden eines Volumens (610), das gereinigt werden kann.

5. Medizinisches Geräteset nach Anspruch 3 oder 4, wobei ein Innenteil (604) der Dichtfläche, der sich in einem Abstand befindet, der größer als der zweite Abstand ist, und eine Innenfläche (606) der Dichtungshülse zum Bilden eines trockenen Volumens (608) funktionell sind.

6. Medizinisches Geräteset nach Anspruch 3, 4 oder 5, wobei der Dichtungshohlraum ferner eine abgeschrägte Oberfläche (300) in Verbindung mit der Dichtfläche aufweist, wobei die abgeschrägte Oberfläche eine Erweiterung des Dichtungshohlraums bildet, wobei die Erweiterung des Dichtungshohlraums sich über einen dritten Abstand (304) in den Dichtungshohlraum erstreckt, wobei der dritte Abstand kleiner als der erste Abstand ist.

7. Medizinisches Geräteset nach Anspruch 2, 3, 4, 5 oder 6, wobei die wenigstens eine erste elektrische Verbindung eine einzelne erste elektrische Verbindung ist, wobei die zweite elektrische Verbindung für jede erste elektrische Verbindung eine einzelne zweite elektrische Verbindung ist, wobei der erste Verbinder und der zweite Verbinder eine koaxiale Verbinderanordnung bilden.

8. Medizinisches Geräteset nach einem der vorhergehenden Ansprüche, wobei die Einklemmfläche ein konisches Profil (214) hat.

9. Medizinisches Geräteset nach einem der vorhergehenden Ansprüche, wobei die Dichtfläche ein Profil mit zwei parallelen Oberflächen (306, 308) hat.

10. Medizinisches Geräteset nach einem der vorhergehenden Ansprüche, wobei die flexible Spitzenregion eine konvexe Oberfläche (406) aufweist, die zur Anlage an der Dichtfläche ausgeführt ist.

11. Medizinisches Geräteset nach einem der vorhergehenden Ansprüche, wobei die konvexe Oberfläche ringförmig ist und wobei die konvexe Oberfläche an einem distalen Ende (408) der Dichtungshülse angebracht ist, wobei die Dichtungshülse einen Außendurchmesser (410) hat, wobei die konvexe Oberfläche einen Dichtungsdurchmesser (412) hat, der größer als der Außendurchmesser ist.

12. Medizinisches Geräteset nach einem der vorhergehenden Ansprüche, wobei die Dichtungshülse ein beliebiges der Folgenden hat: eine Kreissymmetrie und ein oval-förmiges Profil.

13. Medizinisches Geräteset nach einem der vorhergehenden Ansprüche, wobei der erste Verbinder ein erstes Schnappteil (700) aufweist, wobei die Dichtungskappe ein zweites Schnappteil (702) aufweist, wobei das erste Schnappteil und das zweite Schnappteil zum Bilden einer Schnappverbindung (704) zum Zusammenhalten des ersten Verbinders und der Dichtungskappe funktionell sind.

14. Medizinisches Geräteset nach einem der vorhergehenden Ansprüche, wobei das Sensorsystem ein transkutaner Sensor (916) ist und wobei das medizinische Instrument ein Steuerteil (918) eines transkutanen Sensorsystems (910) ist.

15. Verfahren zum Reinigen eines ersten Verbinders (200, 946, 948) eines medizinischen Instruments (918) unter Verwendung einer Dichtungskappe (400), wobei der erste Verbinder wenigstens eine erste elektrische Verbindung (204) aufweist, wobei der erste Verbinder ferner einen Dichtungshohlraum (212) aufweist, der die wenigstens eine erste elektrische Verbindung wenigstens teilweise umgibt, wobei der Dichtungshohlraum eine Dichtfläche (114) und eine Einklemmfläche (214) aufweist, wobei die Dichtungskappe eine Dichtungshülse (401) mit einem inneren Hohlraum (402) aufweist, wobei die Dichtungshülse eine flexible Spitzenregion (404) hat, wobei die Einklemmfläche funktionell ist zum Zwingen der flexiblen Spitze gegen die Dichtfläche, um eine flexible Spitzenabdichtung (600) zu bilden, welche die wenigstens eine erste elektrische Verbindung innerhalb der Dichtungshülse dicht einschließt, wobei das Verfahren die folgenden Schritte aufweist:
- Aufnehmen (800) des mit einem zweiten Verbinder (202) zusammengefügten ersten Verbinders, wobei der zweite Verbinder für jede erste elektrische Verbindung eine zweite elektrische Verbindung (206) aufweist, wobei die zweite elektrische Verbindung zum Bilden einer elektrischen Verbindung mit ihrer ersten elektrischen Verbindung funktionell ist, wobei der zweite Verbinder zum Verbinden mit dem ersten Verbinder funktionell ist, wobei der zweite Verbinder ein Dichtungselement (208) zum Bilden eines O-Dichtrings (218) an der Dichtfläche aufweist, wobei der Dichtungshohlraum ferner eine Öffnung (216) des Dichtungshohlraums aufweist, wobei die Öffnung funktionell ist zum Aufnehmen der Dichtungshülse, wobei das Dichtungselement funktionell ist zum Bilden des O-Dichtrings in einem ersten Abstand (220) von der Öffnung, wenn der zweite Verbinder und der erste Verbinder zusammengefügt werden, wobei die flexible Spitze zum Bilden der flexiblen Spitzenabdichtung in einem zweiten Abstand (602) von der Öffnung funktionell ist und wobei der zweite Abstand größer als der erste Abstand ist,
- Entfernen (802) des zweiten Verbinders von dem ersten Verbinder,
- Einfügen (804) der Dichtungskappe in den ersten Verbinder,
- Reinigen (806) des zweiten Verbinders mit einem Reinigungsfluid und
- Entfernen (808) der Dichtungskappe von dem ersten Verbinder.

## Revendications

1. Dispositif médical en kit comprenant :
- un instrument médical (918) avec un premier connecteur (200, 946, 948), dans lequel le premier connecteur comprend au moins une première connexion électrique (204), dans lequel le premier connecteur comprend en outre une cavité d'étanchéité (212) entourant au moins partiellement la au moins une première connexion électrique, dans lequel la cavité d'étanchéité comprend une surface d'étanchéité (114) et une surface de calage (214) ; et
- un manchon d'étanchéité (400), dans lequel le manchon d'étanchéité comprend un tube de scellement (401) avec une cavité interne (402), dans lequel le tube de scellement a une zone d'extrémité flexible (404), dans lequel la surface de calage est utilisable pour forcer l'extrémité flexible contre la surface d'étanchéité afin de former un joint d'extrémité flexible (600) qui rend étanche la au moins une première connexion électrique dans le tube de scellement.

2. Dispositif médical en kit selon la revendication 1, dans lequel le dispositif médical en kit comprend en outre un système capteur (916) avec un second connecteur (202, 914), dans lequel le second connecteur comprend une seconde connexion électrique (206) pour chaque première connexion électrique, dans lequel la seconde connexion électrique est utilisable pour former une connexion électrique avec sa première connexion électrique, dans lequel le second connecteur est utilisable pour se connecter au premier connecteur, dans lequel le second connecteur comprend un élément d'étanchéité (208) pour former un joint d'étanchéité en forme d'anneau (218) sur la surface d'étanchéité.

3. Dispositif médical en kit selon la revendication 2, dans lequel la cavité d'étanchéité comprend en outre une ouverture (216) dans la cavité d'étanchéité, dans lequel l'ouverture est utilisable pour recevoir le tube de scellement, dans lequel le second connecteur est utilisable pour former le joint d'étanchéité en forme d'anneau à une première distance (220) de l'ouverture lorsque le second connecteur et le premier connecteur sont assemblés, dans lequel l'extrémité flexible est utilisable pour former le joint d'extrémité flexible à une seconde distance (602) de l'ouverture, et dans lequel la seconde distance est supérieure à la première distance.

4. Dispositif médical en kit selon la revendication 3, dans lequel une zone externe (614) de la surface d'étanchéité entre l'ouverture et la seconde distance et une surface extérieure (612) du tube de scellement est utilisable pour former un volume pouvant être nettoyé (610).

5. Dispositif médical en kit selon les revendications 3 ou 4, dans lequel une zone interne (604) de la surface d'étanchéité située à une distance supérieure à la seconde distance et une surface interne (606) du tube de scellement sont utilisables pour former un volume au sec (608).

6. Dispositif médical en kit selon les revendications 3, 4 ou 5, dans lequel la cavité d'étanchéité comprend en outre une surface en biseau (300) qui est en communication avec la surface d'étanchéité, dans lequel la surface en biseau forme un élargissement de la cavité d'étanchéité, dans lequel l'élargissement de la cavité d'étanchéité se prolonge vers la cavité d'étanchéité en une troisième distance (304), dans lequel la troisième distance est inférieure à la première distance.

7. Dispositif médical en kit selon les revendications 2, 3, 4, 5, ou 6, dans lequel la au moins une première connexion électrique est une première connexion électrique unique, dans lequel la seconde connexion électrique pour chaque première connexion électrique est une seconde connexion électrique unique, dans lequel le premier connecteur et le second connecteur forment un assemblage de connecteurs coaxiaux.

8. Dispositif médical en kit selon l'une quelconque des revendications précédentes, dans lequel la surface de calage a un profil conique (214).

9. Dispositif médical en kit selon l'une quelconque des revendications précédentes, dans lequel la surface d'étanchéité a un profil avec deux surfaces parallèles (306, 308).

10. Dispositif médical en kit selon l'une quelconque des revendications précédentes, dans lequel la zone d'extrémité flexible comprend une surface convexe (406) adaptée pour entrer en contact avec la surface d'étanchéité.

11. Dispositif médical en kit selon l'une quelconque des revendications précédentes, dans lequel la surface convexe est en forme d'anneau, et dans lequel la surface convexe est attachée à une extrémité distale (408) du tube de scellement, dans lequel le tube de scellement a un diamètre externe (410), dans lequel la surface convexe a un diamètre d'étanchéité (412) plus important que le diamètre externe.

12. Dispositif médical en kit selon l'une quelconque des revendications précédentes, dans lequel le tube de scellement a un élément quelconque parmi les suivants : une symétrie circulaire et un profil de forme ovale.

13. Dispositif médical en kit selon l'une quelconque des revendications précédentes, dans lequel le premier connecteur comprend un premier composant de fixation rapide (700), dans lequel le manchon d'étanchéité comprend un second composant de fixation rapide (702), dans lequel le premier composant de fixation rapide et le second composant de fixation rapide sont utilisables pour former une connexion rapide (704) afin de maintenir ensemble le premier connecteur et le manchon d'étanchéité.

14. Dispositif médical en kit selon l'une quelconque des revendications précédentes, dans lequel le système de capteur est un capteur transcutané (916), et dans lequel l'instrument médical est une partie de contrôle (918) d'un système de capteur transcutané (910).

15. Procédé de nettoyage du premier connecteur (200, 946, 948) d'un instrument médical (918) utilisant un manchon d'étanchéité (400), dans lequel le premier connecteur comprend au moins une première connexion électrique (204), dans lequel le premier connecteur comprend en outre une cavité d'étanchéité (212) entourant au moins partiellement la au moins une première connexion électrique, dans lequel la cavité d'étanchéité comprend une surface d'étanchéité (114) et une surface de calage (214), dans lequel le manchon d'étanchéité comprend un tube de scellement (401) avec une cavité interne (402), dans lequel le tube de scellement a une zone d'extrémité flexible (404), dans lequel la surface de calage est utilisable pour forcer l'extrémité flexible contre la surface d'étanchéité afin de former un scellement d'extrémité flexible (600) qui rend étanche la au moins une première connexion électrique à l'intérieur du tube de scellement, dans lequel le procédé comprend les étapes :
- de réception (800) du premier connecteur assemblé au second connecteur (202), dans lequel le second connecteur comprend une seconde connexion électrique (206) pour chaque première connexion électrique, dans lequel la seconde connexion électrique est utilisable pour former une connexion électrique avec sa première connexion électrique, dans lequel le second connecteur est utilisable pour se relier au premier connecteur, dans lequel le second connecteur comprend un élément d'étanchéité (208) pour former un joint d'étanchéité en forme d'anneau (218) sur la surface d'étanchéité, dans lequel la cavité d'étanchéité comprend en outre une ouverture (216) de la cavité d'étanchéité, dans lequel l'ouverture est utilisable pour recevoir le tube de scellement, dans lequel l'élément d'étanchéité est utilisable pour former un joint d'étanchéité en forme d'anneau à une première distance (220) à partir de l'ouverture lorsque le second connecteur et le premier connecteur sont assemblés, dans lequel l'extrémité flexible est utilisable pour former un joint d'extrémité flexible à une seconde distance (602) à partir de l'ouverture, et dans lequel la seconde distance est supérieure à la première distance ;
- d'enlèvement (802) du second connecteur d'avec le premier connecteur ;
- d'installation (804) du manchon d'étanchéité dans le premier connecteur ;
- de nettoyage (806) du second connecteur par un fluide de nettoyage ; et
- d'enlèvement (808) du manchon d'étanchéité d'avec le premier connecteur.
